(19) Europäisches Patentamt
European Patent Office
Office européen des brevets

(11) Publication number: **0 258 981 A2**

# (12) EUROPEAN PATENT APPLICATION

(21) Application number: **87306096.6**

(51) Int. Cl.4: **A01N 43/84** , A01N 43/40

(22) Date of filing: **10.07.87**

(30) Priority: **15.08.86 GB 8619913**
**08.10.86 GB 8624125**

(43) Date of publication of application:
**09.03.88 Bulletin 88/10**

(84) Designated Contracting States:
**AT BE CH DE ES FR GB GR IT LI LU NL SE**

(71) Applicant: **IMPERIAL CHEMICAL INDUSTRIES PLC**
**Imperial Chemical House Millbank**
**London SW1P 3JF(GB)**

Applicant: **KAKEN PHARMACEUTICAL CO., LTD.**
**No. 28-8, 2-chome, Honkomagome**
**Bunkyo-Ku**
**Tokyo 113(JP)**

(72) Inventor: **Brown, Mark Carnegie**
**45 Holmsdale**
**Crown Wood Bracknell Berkshire(GB)**
Inventor: **Elliott, Alison Clare**
**14 Huntington Close**
**Lower Earley Near Reading Berkshire(GB)**
Inventor: **Worthington, Paul Anthony**
**4 Oakhurst Road Maidenhead**
**Court Park Maidenhead Berkshire(GB)**

(74) Representative: **Alner, Henry Giveen Hamilton et al**
**Imperial Chemical Industries PLC Legal Department: Patents P.O. Box 6 Bessemer Road**
**Welwyn Garden City Herts, AL7 1HD(GB)**

## (54) Process for combating fungi in agriculture.

(57) A method of combating fungi and bacteria in agriculture comprising applying to a plant, to seed of a plant, or to the locus of the plant or seed a thiophene derivative represented by general formula :

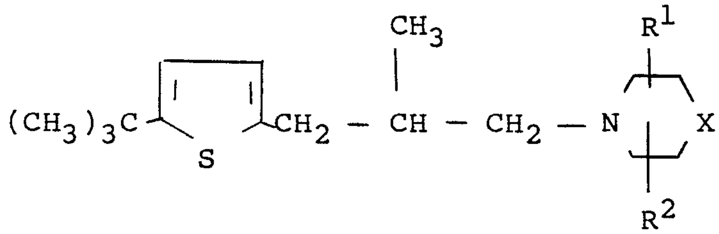

wherein R2 and R2 each represents a hydrogen atom or an alkyl group having 1 to 4 carbon atoms and X represents a methylene group or an oxygen atom, or an acid addition salt thereof.

EP 0 258 981 A2

# PROCESS FOR COMBATING FUNGI IN AGRICULTURE

This invention relates to a process for combating fungi and bacteria, especially fungal and bacterial infections in plants; and to fungicidal and bactericidal compositions useful in agriculture.

The invention provides a process for combating fungi and bacteria which comprises applying to a plant to seed of a plant, or to the locus of the plant or seed a thiophene derivative having the general formula (I) :

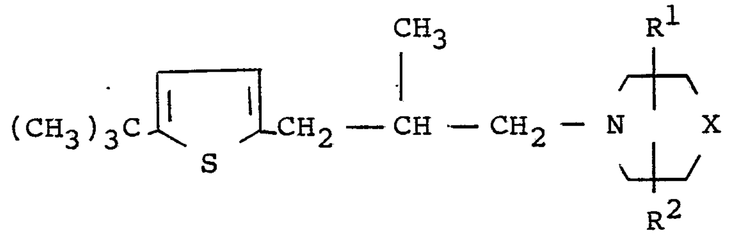

(I)

wherein $R_1$ and $R_2$ each represents a hydrogen atom or an alkyl group having 1 to 4 carbon atoms and X represents a methylene group or an oxygen atom, or an acid addition salt thereof.

The alkyl groups may be straight or branched chain groups having 1 to 6, eg, 1 to 4, carbon atoms; examples are methyl, ethyl, propyl (n-or iso-propyl) and butyl (n -, sec-, iso or t-butyl) and methyl is especially preferred.

The salts can be salts with inorganic or organic acids eg, hydrochloric, hydrobromic, nitric, sulphuric, oxalic, acetic, tartaric, 4-toluene-sulphonic or methanesulphonic acid.

Examples of active compounds for use in the invention process include 2-tert-butyl-5-(2-methyl-3-piperidino-propyl)thiophene, 2-tert-butyl-5-[2-methyl-3-(2-methylpiperidino)propyl]thiophene, 2-tert-butyl-5-[2-methyl-3-(3-methylpiperidino)propyl]thiopene, 2-tert-butyl-5-[2-methyl-3-(4-methylpiperidino)propyl]-thiopene, 2-tert-butyl-5-[3-(3,5-dimethylpiperidino)-2-methylpropylJthiophene, 2-tert -butyl-5-(2-methyl-3-morpholinopropyl) thiophene, 2-tert-butyl-5-[3-(2,6-dimethylmorpholino)-2-methylpropyl]thiophene and their acid addition salts

The invention further provides a composition for combating fungi and bacteria in agriculture which comprises as an active ingredient, a thiophene derivative as defined in any of the preceding paragraphs.

The thiophene derivatives are readily manufactured by reduction of the thienylketone compound (II) of general formula :

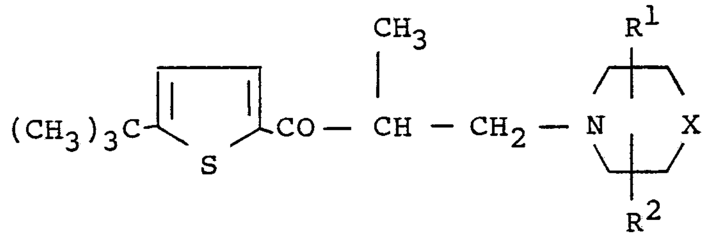

(II)

(where $R_1$ and $R_2$ represent a hydrogen atom or an alkyl group having 1 to 4 carbon atoms, respectively, X, a methylene group or an oxygen atoms) by the Clemmensen technique [E.L. Martin; Organic Reactions Vol 1, 155 (1942)]. The said Clemmensen reduction reaction normally takes place in hydrochloric acid as solvent and the concentrations of the hydrochloric acid used are in the range of 5-35%, preferably, 20-30%. The zinc amalgam used is preferably prepared before use, and 1-3 parts of zinc dust is used to one part of the raw material thienylketone compound (II). The reaction is preferably carried out by stirring the mixture for 15 minutes - 48 hours at ambient temperature or solvent reflux temperature. The addition of 35% hydrochloric acid in about 1-5 portions at appropriate intervals is preferred. The resultant thiopene derivative (I) can be isolated by the ordinary technique and clean-up is achieved by vaccuum distillation, recrystallisation, chromatography and other techniques. Where necessary, the free bases of the derivative can be converted to acid addition salts by standard techniques.

The thienylketone compound (II) used as a starting material in the reduction reaction is a new compound which can be prepared by, for example, the Mannich reaction [F.F. Blicke; Organic Reactions Vol 1, 308 (1942)] :

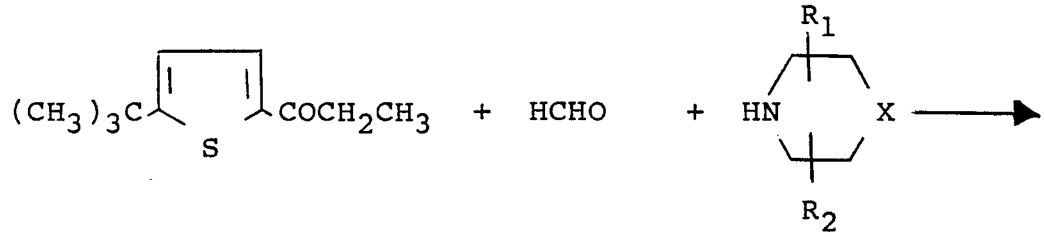

(II)

.HCl

(III) (IV)

(where $R_1$, $R_2$ and X represent the same atoms or groups as described previously). This reaction is preferably carried out by agitation and reflux of 2-tert -butyl-5-propionylthiopene (II), 35% formaldehyde solution and the amine compound (IV) in ethanol for 1-10 hours following the ordinary technique for the Mannich reaction. The resultant thienylketone compound (II) can be isolated and purified by the usual methods.

The fungicidal compositions of the invention are active against, and therefore the invention process is useful in combating, the following diseases Puccinia reconita, Puccinia striiformis and other rusts on wheat, Puccinia hordei, Puccinia striiformis and other rusts on barley, and rusts on other hosts eg, coffee, first trees especially apples, vegetables and ornamental plants Erysiphe graminis(powdery mildew) on barley and wheat and other powdery mildews on various hosts such as Sphaerotheca fuliginea on cucurbits (eg, cucumber), Podosphaera leucotricha on apples and Uncinula necator on vines Helminthosporium spp. and Rhynchosporium spp. on cereals Cercospora arachidicola on peanuts and other Cercospora species on for example sugar beet, bananas and soya beans Botrytis cinerea (grey mould) on tomatoes, strawberries, vines and other hosts Venturia inaequalis (scab) on apples.

Some of the compounds have also shown a broad range of activities against fungi in vitro. They have activity against various post-harvest diseases on fruit (eg, Penicillium digatatum and italicum on oranges and Gloeosporium musarum on bananas). Further some of the compounds are active as seed dressings against:

Fusarium spp., Septoria spp., Tilletia spp. (i.e. bunt, a seed borne disease of wheat), Ustilago spp. and Helminthosporium spp. on cereals, Rhizoctonia solani on cotton and Corticiumsasakii on rice.

The compounds can move acropetally in the plant tissue. Moreover, the compounds can be volatile enough to be active in the vapour phase against fungi on the plant.

They may also be useful as industrial (as opposed to agricultural) fungicides, eg, in the prevention of fungal attack on wood, hides, leather and especially paint films.

The compounds also display plant anti-bacterial activity, eg, against the disease Xanthomonas oryzae (bacterial blight of rice), and the present invention therefore includes a process of treating plants to combat bacterial diseases.

The compounds may be used as such for fungicidal purposes but are more conveniently formulated into compositions for such usage. The invention thus provides a fungicidal or plant growth regulating composition comprising a compound of general formula (I) as hereinbefore defined, or an acid addition salt thereof.

The compounds and their acid addition salts can be applied in a number of ways, for example they can be applied, formulated or unformulated, directly to the foliage of a plant, or they can be applied also to bushes and trees, to seeds or to other media in which plants, bushes or trees are growing or are to be planted, or they can be sprayed on, dusted on or applied as a cream or paste formulation, or they can be applied as a vapour; or as slow release granules. Application can be to any part of the plant, bush or tree,

for example to the foliage, stems, branches or roots, or to soil surrounding the roots, or to the seed before it is planted; or to the soil generally, to paddy water or to hydroponic culture systems. The invention compounds may also be injected into plants or trees and they may also be sprayed onto vegetation using electrodynamic spraying techniques.

The term "plant" as used herein includes seedlings, bushes and trees. Furthermore, the fungicidal method of the invention includes preventative, protectant, prophylactic and eradicant treatment.

The compounds are preferably used for agricultural and horticultural purposes in the form of a composition. The type of composition used in any instance will depend upon the particular purpose envisaged.

The compositions may be in the form of dusting powders or granules comprising the active ingredient and a solid diluent or carrier, for example fillers such as kaoline, bentonite, kieselguhr, dolomite, calcium carbonate, talc, powdered magnesia, Fuller's earth, gypsum, Hewitt's earth, diatomaceous earth and China clay. Such granules can be preformed granules suitable for application to the soil without further treatment. These granules can be made either by impregnating pellets of filler with the active ingredient or by pelleting a mixture of the active ingredient and powdered filler. Compositions for dressing seed, for example, may comprise an agent (for example a mineral oil) for assisting the adhesion of the composition to the seed; alternatively the active ingredient can be formulated for seed dressing purposes using an organic solvent (for example N-methylpyrrolidone or dimethylformamide).

The compositions may also be in the form of dispersible powders, granules or grains comprising a wetting agent to facilitate the dispersion in liquids of the powder or grains which may contain also fillers and suspending agents.

The aqueous dispersions or emulsions may be prepared by dissolving the active ingredient(s) in an organic solvent optionally containing wetting, dispersing or emulsifying agent(s) and then adding the mixture to water which may also contain wetting, dispersing or emulsifying agent(s). Suitable organic solvents are ethylene dichloride, isopropyl alcohol, propylene glycol, diacetone alcohol, toluene, kerosene, methylnaphthalene, the xylenes, trichloroethylene, furfuryl alcohol, tetrahydrofurfuryl alcohol, and glycol ethers (eg, 2-ethoxyethanol and 2-butoxyethanol).

The compositions to be used as sprays may also be in the form of aerosols wherin the formulation is held in a container under pressure in the presence of a propellant, eg, fluorotrichloromethane or dichlorodifluoromethane.

The compounds can be mixed in the dry state with a pyrotechnic mixture to form a composition suitable for generating in enclosed spaces a smoke containing the compounds.

Alternatively, the compounds may be used in a microencapsulated form. They may also be formulated in biodegradable polymeric formulations to obtain a slow, controlled release of the active substance.

By including suitable additives, for example additives for improving the distrubution, adhesive power and resistance to rain on treated surfaces, the different compositions can be better adapted for various utilities.

The compounds can be used as mixtures with fertilizers (eg, nitrogen-, potassium-or phosphorus-containing fertilizers). Compositions comprising only granules of fertilizer incorporating, for example coated with, the compound are preferred. Such granules suitable contian up to 25% by weight of the compound. The invention therefore also provides a fertilizer composition comprising the compound of general formula (I) or a salt or metal complex thereof.

The compositions may also be in the form of liquid preparations for use as dips or sprays which are generally aqueous dispersions or emulsions containing the active ingredient in the presence of one or more surfactants eg, wetting agent(s), dispersing agent(s), emulsifying agent(s) or suspending agent(s); or which are spray formulations of the kind suitable for use in electrodynamic spraying techniques. The foregoing agents can be cationic, anionic or non-ionic agents. Suitable cationic agents are quaternary ammonium compounds, for example cetyltrimethyl-ammonium bromide.

Suitable anionic agents are soaps, salts or aliphatic monoesters of sulphuric acid (for example sodium lauryl sulphate), and salts of sulphonated aromatic compounds (for example sodium dodecylbenzenesulphonate, sodium, calcium or ammonium lignosulphonate, butylnaphthalene sulphonate, and a mixture of sodium diisopropyl-and triisopropyl-naphthalene sulphonates).

Suitable non-ionic agents are the condensation products of ethylene oxide with fatty alcohols such as olely or cetyl alcohol, or with alkyl phenols such as octyl-or nonyl-phenol and octylcresol. Other non-ionic agents are the partial esters derived from long chain fatty acids and hexitol anhydrides, the condensation products of the said partial esters and ethylene oxide, and the lecithins. Suitable suspending agents are hydrophilic colloids (for example polyvinylpyrrolidone and sodium carboxymethylcellulose), and the vegetable gums (for example gum acacia and gum tragacanth).

The compositions for use as aqueous dispersions or emulsions are generally supplied in the form of a concentrate containing a high proportion of the active ingredient(s), and the concentrate is to be diluted with water before use. These concentrates often should be able to withstand storage for prolonged periods and after such storage be capable of dilution with water in order to form aqueous preparations which remain homogeneous for a sufficient time to enable them to be applied by conventional and electrodynamic spray equipment. The concentrates may conveniently contain up to 95%, suitably 10-85%, for example 25-60%, by weight of the active ingredient(s). These concentrates suitably contain organic acids (eg, alkaryl or aryl sulphonic acids such as xylenesulphonic acid or dodecyl benzenesulphonic acid) since the presence of such acids can increase the solubility of the active ingredient(s) in the polar solvents often used in the concentrates. The concentrates suitably contain also a high proportion of surfactants so that sufficiently stable emulsions in water can be obtained. After dilution to form aqueous preparations, such preparations may contain varying amounts of the active ingredient(s) depending upon the intended purpose, but an aqueous preparation containing 0.0005% or 0.01% to 10% by weight of active ingredient(s) may be used.

The compositions of this invention can comprise also other compound(s) having biological activity, eg, compounds having similar or complementary fungicidal or plant growth activity or compounds having plant growth regulating, herbicidal or insecticidal activity.

The other fungicidal compound can be, for example, one which is capable of combating ear diseases of cereals (eg, wheat) such as Septoria, Gibberella and Helminthosporium spp., seed and soil borne diseases and downy and powdery mildews on grapes and powdery mildew and scab on apple etc. These mixtures of fungicides can have a broader spectrum of activity than the compound of general formula (I) alone; further the other fungicide can have a synergistic effect on the fungicidal activity of the compound of general formula (I). Examples of the other fungicidal compound are carbendazim, benomyl, thiophanate-methyl, thiabendazole, fuberidazole, etridazole, dichlofluanid, cymoxanil, oxadixyl, ofurace, metalaxyl, furalaxyl, benalaxyl, fosetyl-aluminium, fenarimol, iprodione, procymidione, vinclozolin, penconazole, myclobutanil, R0151297, pyrazophos, ethirimol, ditalimfos, tridemorph, triforine, nuarimol, triazbutyl, guazatine, triacetate salt of l,l'iminodi(octamethylene)-diguanidine, propiconazole, prochloraz, flutriafol, hexaconazole ie. the chemical 1-(1,2,4-triazol-1-yl)-2(2,4-dichlorophenyl)hexan-2-ol, (2RS, 3 RS)-2-(4-chlorophenyl)-3-cyclopropyl-1-(IH,1,2,4-triazol-1-yl)butan2-ol, (RS)-1-(4-chlorophenyl)-4,4,4-dimethyl-3-(IH-1,2,4-triazol-1-ylmethyl)-pentan-3-ol, DPX H6573(1-((bis-4-fluorophenyl)methylsilyl)methyl)IH-1,2,4-triazole, triadimefon, triadimenol, diclobutrazol, fenpropimorph, fenpropidin, chlorozolinate, diniconazole, imazalil, fenfuram, carboxin, oxycarboxin, methfuroxam, dodemorph, BAS 454, blasticidin S, kasugamycin, edifenphos, kitazin P, cycloheximide, phthalide, probenazole, isoprothiolane, tricyclazole, pyroquilon, 4-chloro-N-(cyano(ethoxy)methyl)-benzamide, chlorbenzthiazone, neoasozin, polyoxin D, validamycin A, mepronil, flutolanil, pencycuron, diclomezine, phenazin oxide, nickel dimethyldithiocarbamate, techlofthalam, bitertanol, bupirimate, etaconazole, streptomycin, cypofuram, biloxazol, quinomethionate, dimethirimol, 1-(2-cyano-2-methoxyimino-acetyl)-3-ethyl urea, fenapanil, tolclofosmethyl, pyroxyfur, polyram, maneb, mancozeb, captafol, chlorothalonil, anilazine, thiram, captan, folpet, zineb, propineb, sulphur, dinocap, binapacryl, nitrothalisopropyl, dodine, dithianon, fentin hydroxide, fentin acetate, tecnazene, quintozene, dichloran, copper containing compounds such as copper oxychloride, copper sulphate and Bordeaux mixture, and organomercury compounds.

The compounds of general formula (I) can be mixed with soil, peat or other rooting media for the protection of plants against seed-borne, soil-borne or foliar fungal diseases.

Suitable insecticides which may be incorporated in the composition of the invention include pirimicarb, dimethoate, demeton-s-methyl, formothion, carbaryl, isoprocarb, XMC, BPMC, carbofuran, carbosulfan, diazinon, fenthion, fenitrothion, phenthoate, chlorpyrifos, isoxathion, propaphos, monocrotophas, buprofezin, ethroproxyfen and cycloprothrin.

Suitable growth regulating compounds are compounds which control weeds or seedhead formation, or selectively control the growth of less desirable plants (eg, grasses).

Examples of suitable plant growth regulating compounds for use with the invention compounds are the gibberellins (eg, $GA_3$, $GA_4$ or $GA_7$), the auxins (eg, indoleacetic acid, indolebutyric acid, naphthoxyacetic acid or naphthylacetic acid), the cytokinins (eg, kinetin, diphenylurea, benzimidazole, benzyladenine or benzylaminopurine), phenoxyacetic acids (eg, 2,4-D or MCPA), substituted benzoic acids (eg, triiodobenzoic acid), morphactins (eg, chlorfluoroecol), maleic hydrazide, glyphosate, glyphosine, long chain fatty alcohols and acids, dikegulac, paclobutrazol, flurprimidol, fluoridamid, mefluidide, substituted quaternary ammonium and phosphonium compounds (eg, chloromequat chlorphonium or mepiquatchloride), ethephon, carbetamide, methyl-3,6-dichloroanisate, daminozide, asulam, abscisic acid, isopyrimol, 1-(4-chlorophenyl)-4,6-dimethyl-2-oxo-1,2-dihydropyridine-3-carboxylic acid, hydroxybenzonitriles (eg, bromoxynil), difenzoquat, benzoylprop-ethyl 3,6-dichloropicolinic acid, fenpentezol, inabenfide, triapenthenol and tecnazene.

The following Examples illustrate the invention. Examples Nos. 1-7 and 1A to 7A illustrate the preparation of the active compounds used in the invention compositions and process. Examples Nos 8-17 illustrate compositions according to the invention. Example 18 illustrates the process of the invention.

EXAMPLE 1

A mixture of 2-tert-butyl-5-propionylthiophene (2.94g), 3,5-dimethylpiperidine hydrochloride (3.34g), 35% formaldehyde solution in water (3.86g), ethanol (5ml) and one drop of concentrated hydrochloric acid is refluxed with agitation for six hours. After the completion of the reaction, the mixture is cooled with ice-cold water, and the white solid material deposited is filtered off and washed with small amounts of water. Recrystallisation of the resultant white solid material from a hexane-acetone solution gives 2-tert-butyl-5-[3-(3,5-dimethylpiperidino)-2-methylpropionyl]thiophene hydrochloride as white crystalline plates (3.84g) with melting point between 188 - 190°C (decomposition).

EXAMPLES 2 - 7

The compounds given in the following table (Table I) were obtained by using the same procedures as given in Example 1 except that new starting materials were used in Examples 2-7 instead of 3,5-dimethylpiperidine hydrochloride.

TABLE I

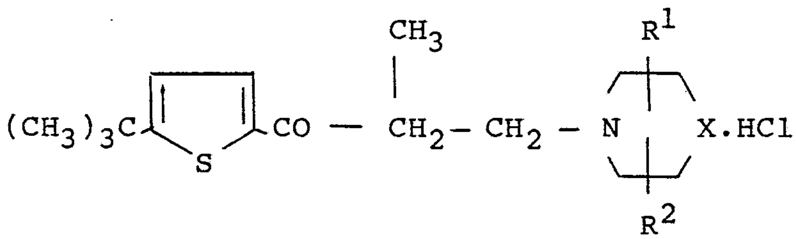

| EXAMPLE | $R_1$ | $R_2$ | X | MELTING POINT (°C) |
|---|---|---|---|---|
| 2 | H | H | $CH_2$ | 187-188 (decomposes) |
| 3 | $2\text{-}CH_3$ | H | $CH_2$ | oily substance |
| 4 | $3\text{-}CH_3$ | H | $CH_2$ | 196.5-197.5 (decomposes) |
| 5 | $4\text{-}CH_3$ | H | $CH_2$ | 192.5-193.5 (decomposes) |

TABLE I CONTINUED

| EXAMPLE | $R_1$ | $R_2$ | X | MELTING POINT (°C) |
|---|---|---|---|---|
| 6 | H | H | O | 178-179 (decomposes) |
| 7 | 2-$CH_3$ | 6-$CH_3$ | O | 177-178 (decomposes) |

The major signals obtained in NMR and mass spectrometry of the compounds of Examples 1 - 7 are shown in the following table (Table II).

TABLE II

| Compound of | NMR (in $CDCl_3$), Delta Value, PPM | Mass Spectrometry: m/e |
|---|---|---|
| Example 1 | 1.42 (s, 9H)<br>6.92 (d, J=4Hz, 1H)<br>7.90 (d, J=4Hz, 1H) | 126 (base peak), 321 ($M^+$) |
| Example 2 | 1.42 (s, 9H)<br>6.93 (d, J=4Hz, 1H)<br>7.89 (d, J=4Hz, 1H) | 98 (base peak), 293 ($M^+$) |
| Example 3 | 1.42 (s, 9H)<br>6.96 (s, J=4Hz, 1H)<br>7.93 (d, J=4Hz, 1H) | 112 (base peak), 307 ($M^+$) |
| Example 4 | 1.42 (s, 9H)<br>6.90 (d, J=4Hz, 1H)<br>7.89 (d, J=4Hz, 1H) | 112 (base peak), 307 ($M^+$) |
| Example 5 | 1.42 (s, 9H)<br>6.93 (d, J=4Hz, 1H)<br>7.90 (d, J=4Hz, 1H) | 112 (base peak), 307 ($M^+$) |

TABLE II CONTINUED

| Compound of | NMR (in $CDCl_3$) Delta Value, PPM | Name Spectrometry: m/e |
|---|---|---|
| Example 6 | 1.40 (s, 9H)<br>6.95 (d, J=4Hz, 1H)<br>7.90 (d, J=4Hz, 1H) | 100 (base peak), 295 ($M^+$) |
| Example 7 | 1.42 (s, 9H)<br>6.97 (d, J=4Hz, 1H)<br>7.93 (d, J=4Hz, 1H) | 128 (base peak), 323 ($M^+$) |

EXAMPLE IA

The freshly prepared zinc amalgam (which is prepared by adding mercury (II) chloride (0.21g) to a mixture of zinc dust (2.13g) and 25% hydrochloric acid (4ml) while agitating the mixture and decanting off the supernatant when two layers separated after a while) is admixed with 2-tert-butyl-5-[3-(3,5-dimethylpiperidino)-2-methylpropionyl]thiophene hydrochloride (1.79g) obtained in Example 1 and 25% hydrochloric acid (8ml) and then refluxed for 24 hours with agitation. During the course of the reaction, 35% hydrochloric acid (2ml) is added twice at 8-hour intervals. After completion of the reaction, the reaction solution is diluted with water, alkalised by the addition of caustic soda and subsequently extracted with benzene. The benzene layer is washed with water, dried with anhydrous sodium sulphate and vaporisation of benzene gives oily 2-tert-butyl-5-[3-(3,5dimethylpiperidino)-2-methylpropyl]-thiophene (1.29g). The resultant oily substance is dissolved in carbon tetrachloride. Dry hydrogen chloride gas is blown in. The deposited white solid is filtered off and recrystallised from an acetone-hexane mixture to obtain 2-tert-butyl-5-[3,5-dimethylpiperidino)-2-methylpropyl]thiophene hydrochloride as white crystalline plates with melting point in the 192-195°C range.

EXAMPLES 2A - 7A

The compounds given in the following table (Table III) were obtained by using the same procedures as given in Example IA, except that new starting materials were used in Examples 2A - 7A instead of 2-tert-butyl-5-[3(3,5-dimethylpiperidino)-2-methylpropionyl]thiophene hydrochloride.

TABLE III

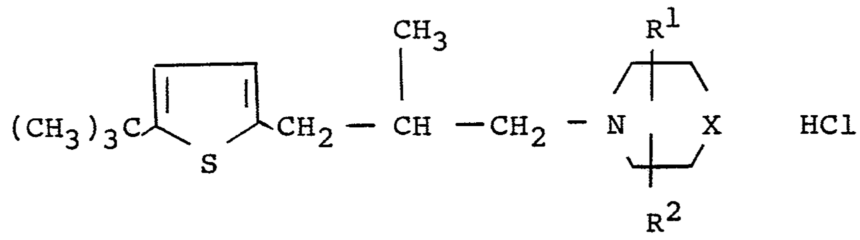

| EXAMPLE | $R_1$ | $R_2$ | X | MELTING POINT (°C) |
|---|---|---|---|---|
| 2A | H | H | $CH_2$ | oily substance |
| 3A | $2-CH_3$ | H | $CH_2$ | oily substance |
| 4A | $3-CH_3$ | H | $CH_2$ | 133-135 (decomposes) |
| 5A | $4-CH_3$ | H | $CH_2$ | oily substance |
| 6A | H | H | O | 178-180 (decomposes) |
| 7A | $2-CH_3$ | $6-CH_3$ | O | 206-207 (decomposes) |

The major signals obtained in NMR and mass spedtrometry of the compounds of Examples 1A - 7A are shown in the following table (Table IV).

TABLE IV

| Compound of | NMR (in $CDCl_3$), Delta Value, PPM | Mass Spectrometry: m/e |
|---|---|---|
| Example 1A | 1.32 (s, 9H), 6.60 (s, 2H) | 126 (base peak), 307 ($M^+$) |
| Example 2A | 1.33 (s, 9H), 6.63 (s, 2H) | 98 (base peak), 279 ($M^+$) |
| Example 3A | 1.33 (s, 9H), 6.58 (s, 2H) | 112 (base peak), 293 ($M^+$) |
| Example 4A | 1.33 (s, 9H), 6.60 (s, 2H) | 112 (base peak), 293 ($M^+$) |
| Example 5A | 1.32 (s, 9H), 6.58 (s, 2H) | 112 (base peak), 293 ($M^+$) |
| Example 6A | 1.33 (s, 9H), 6.59 (s, 2H) | 100 (base peak), 281 ($M^+$) |
| Example 7A | 1.37 (s, 9H), 6.62 (s, 2H) | 128 (base peak), 309 ($M^+$) |

EXAMPLE 8

An emulsifiable concentrate was made up by mixing the ingredients, and stirring the mixture until all the constituents were dissolved.

Compound of Example 1 10%
Ethylene dichloride 40%
Calcium dodecylbenzenesulphate 5%
"Lubrol" L 10%
"Aromasol" H 35%

EXAMPLE 9

A composition in the form of grains readily dispersible in a liquid, eg, water, was prepared by grinding together the first three ingredients in the presence of added water and then mixing in the sodium acetate. The resultant mixture was dried and passed through a British Standard mesh sieve, size 44-100, to obtain the desired size of grains.

Compound of Example 2 50% "Dispersol" T 25%
"Lubrol" APN5 1.5%
Sodium acetate 23.5%

EXAMPLE 10

The ingredients were all ground together to produce a powder formulation readily dispersible in liquids.
Compound of Example 3 45%
"Dispersol" T 5%
"Lissapol" NX 0.5%
"Cellofas" B600 2%
Sodium acetate 47.5%

EXAMPLE 11

The active ingredient was dissolved in a solvent and the resultant liquid was sprayed on to the granules of China clay. The solvent was then allowed to evaporate to produce a granular composition.
Compound of Example 4 5%
China clay 95%

EXAMPLE 12

A composition suitable for use as a seed dressing was prepared by mixing the three ingredients.
Compound of Example 5 50%
Mineral oil 2%
China clay 48%

EXAMPLE 13

A dusting powder was prepared by mixing the active ingredient with talc.
Compound of Example 6 5%
Talc 95%

EXAMPLE 14

A Col formulation was prepared by ball-milling the constituents set out below and then forming an aqueous suspension of the ground mixture with water.
Compound of Example 7 40%
"Dispersol" T 10%
"Lubrol" APN5 1%
Water 1%

EXAMPLE 15

A dispersible powder formulation was made by mixing together the ingredients set out below and then grinding the mixture until all were thoroughly mixed.

Compound of Example 1A 25%
"Aerosol" OT/B 2%
"Dispersol" A.C. 5%
China clay 28%
Silica 40%

EXAMPLE 16

This Example illustrates the preparation of a dispersible powder formulation. The ingredients were mixed and the mixture then ground in a comminution mill. Compound of Example 2A 25%
"Perminal" BX 1%
"Dispersol" T 5%
Polyvinylpyrrolidone 10%
Silica 25%
China clay 34%

EXAMPLE 17

The ingredients set out below were formulated into a dispersible powder by mixing then grinding the ingredients.

Compound of Example 3A 25%
"Aerosol" OT/B 2%
"Dispersol" A 5%
China clay 68%

In Examples 8 to 17 are the proportions of the ingredients given are by weight.

The compounds set out in Examples 1 to 7 and 1A to 7A were all similarly formulated as specifically described in Examples 8 to 17.

There now follows an explanation of the compositions or substances represented by the various Trade Marks and Trade Names mentioned above.

LUBROL L : a condensate of nonyl phenol (1 mole) with ethylene oxide (13 moles)

AROMASOL H : a solvent mixture of alkylbenzenes

DISPERSOL T & AC : a mixture of sodium sulphate and a condensate of formaldehyde with sodium naphthalene sulphonate

LUBROL APN5 : a condensate of nonyl phenol (1 mole) with naphthalene oxide (5.5 moles)

CELLOFAS B600 : a sodium carboxymethyl cellulose thickener

LISSAPOL NX : a condensate of nonyl phenol (1 mole) with ethylene oxide) (8 moles)

AEROSOL OT/B : dioctyl sodium sulphosuccinate

PERMINAL BX : a sodium alkyl naphthalene sulphonate

EXAMPLE 18

The compounds were tested against a variety of foliar fungal diseases of plants. The technique employed was as follows.

The plants were grown in John Innes Potting Compost (No 1 or 2) in 4 cm diameter minipots). The test compounds were formulated either by bead milling with aqueous Dispersol T or as a solution in acetone or acetone/ethanol which was diluted to the required concentration immediately before use. For the foliage diseases, the formulations (100 ppm active ingredient) were sprayed on to the foliage and applied to the roots of the plants in the soil. The sprays were applied to maximum retention and the root drenches to a final concentration equivalent to approximately 40 ppm a.i./dry soil. Tween 20, to give a final concentration of 0.05%, was added when the sprays were applied to cereals.

For most of the tests the compound was applied to the soil (roots) and to the foliage (by spraying) one or two days before the plant was inoculated with the disease. An exception was the test on Erysiphe graminis in which the plants were inoculated 24 hours before treatment. Foliar pathogens were applied by spray as spore suspensions onto the leaves of test plants. After inoculation, the plants were put into an appropriate environment to allow infection to proceed and then incubated until the disease was ready for assessment. The period between inoculation and assessment varied from four to fourteen days according to the disease and environment.

The disease control was recorded by the following grading :

4 = no disease
3 = trace -5% of disease on untreated plants
2 = 6-25% of disease on untreated plants
1 = 26-95% of disease on untreated plants
0 = 60-100% of diseases on untreated plants

TABLE V

| COMPOUND OF EXAMPLE NUMBER | PUCCINIA RECONDITA (WHEAT) | ERYSIPHE GRAMINIS (BARLEY) | CERCOSPORA ARACHIDICOLA (PEANUT) | VENTURIA INAEQUALIS (APPLE) | XANTHOMONAS ORYZAE (RICE) |
|---|---|---|---|---|---|
| 1A | 3 | 4 | 0 | 0 | 3 |
| 2A | 2 | 4 | 0 | 0 | 0 |
| 4A | 3 | 4 | 0 | 0 | 0 |
| 6A | 0 | 4 | 0 | 0 | 0 |
| 7A | 4 | 4 | 4 | 4 | 3 |

PP 33997
HGHA/CF
03 Jul 87

**Claims**

1. A method of combating fungi and bacteria, which comprises applying to a plant, to seed of a plant, or to the locus of the plant or seed, a thiophene derivative represented by general formula :

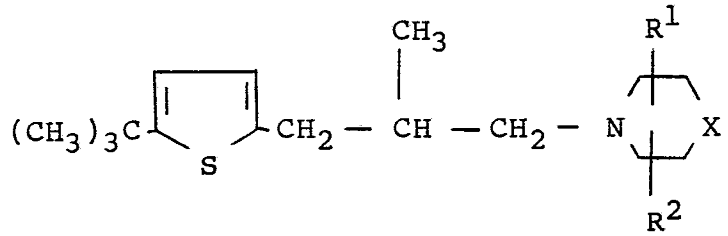

.wherein $R_1$ and $R_2$ each represents a hydrogen atom or an alkyl group having 1 to 4 carbon atoms and X represents a methylene group or an oxygen atom, or an acid addition salt thereof.

2. A method according to claim 1 wherein the thiophene derivative is 2-tert-butyl-5-(2-methyl-3-piperidinopropyl)thiophene.

3. A method according to claim 1 wherein the thiophene derivative is 2-tert-butyl-5-[2-methyl-3-(2-methylpiperidino)propyl]thiophene.

4. A method according to claim 1 wherein the thiophene derivative is 2-tert -butyl-5-[2-methyl-3-(3-methylpiperidino)propyl]thiophene.

5. A method according to claim 1 wherein the thiophene derivative is 2-tert-butyl-5-[2-methyl-3-(4-methylpiperidino)propyl]thiophene.

6. A method according to claim 1 wherein the thiopene derivative is 2-tert-butyl-5-[3-(3,5-dimethylpiperidino)-2-methylpropyl]thiophene.

7. A method according to claim 1 wherein the thiophene derivative is 2-tert-butyl-5-(2-methyl-3-morpholinopropyl)thiophene.

8. A method according to claim 1 wherein the thiophene derivative is 2-tert -butyl-5-[3-(2,6-dimethylmorpholino)-2-methylpropyl]thiophene.

9. A process for combating-plant fungi which comprises applying to plants or seeds, or to their locus.

10. An antifungal and antibacterial composition for use in agriculture comprising, as an active ingredient, a thiophene derivative represented by general formula :

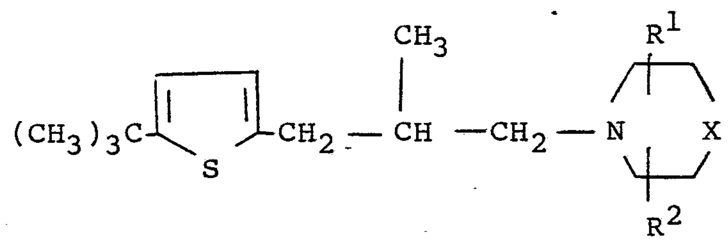

wherein $R_1$ and $R_2$ each represents a hydrogen atom or an alkyl group having 1 to 4 carbon atoms and X represents a methylene group or an oxygen atom, or an acid addition salt thereof.

11. An antifungal composition according to claim 10 wherein the active ingredient is 2-tert-butyl-5-(2-methyl-3-piperidinopropyl)thiophene.

12. An antifungal composition according to claim 10 wherein the active ingredient is 2-tert-butyl-5-[2-methyl-3-(2-methylpiperidino)propyl]thiophene.

13. An antifungal composition according to claim 10 wherein the active ingredient is 2-tert-butyl-5-[2-methyl-3-(3-methylpiperidino)propyl]thiophene.

14. An antifungal composition according to claim 10 wherein the active ingredient is 2-tert-butyl-5-[2-methyl-3-(4-methylpiperidino)propyl]thiophene.

15. An antifungal composition according to claim 10 wherein the active ingredient is 2-tert-butyl-5-[3-(3,5-dimethylpiperidino)-2-methylpropyl]thiophene.

16. An antifungal composition according to claim 10 wherein the active ingredient is 2-tert-butyl-5-(2-methyl-3-morpholinopropyl)thiophene.

17. An antifungal composition according to claim 10 wherein the active ingredient is 2-tert-butyl-5-[3-(2,6-dimethylmorpholino)-2-methylpropyl]thiophene.